# EUROPEAN PATENT SPECIFICATION

(11) **EP 2 791 141 B1**
(45) Date of publication and mention of the grant of the patent: **08.02.2017**
(21) Application number: 12805898.9
(22) Date of filing: 13.12.2012
(51) Int. Cl.: C07D 487/04, A61K 31/519, A61P 35/00

(54) **Tofacitinib mono-tartrate salt**
Mono-Tartratsalz von Tofacitinib
Sel de mono-tartrate de Tofacitinib

(30) Priority: 15.12.2011 US 201161576199 P
(43) Date of publication of application: 22.10.2014
(60) Divisional application: 16202511.8
(73) Proprietor: ratiopharm GmbH, 89079 Ulm (DE)
(72) Inventor: COUTABLE, ludovic, 89077 Ulm (DE); ALBRECHT, Wolfgang, 89075 Ulm (DE); GUSERLE, Richard, 89359 Kötz (DE)
(74) Representative: D Young & Co LLP
(86) International application number: PCT/US2012/069355
(87) International publication number: WO 2013/090490

(56) References cited:
- WO-A1-03/048162
- WO-A1-2012/135338
- WO-A2-2007/012953

## Description

### Field of the Invention

The present invention encompasses amorphous Tofacitinib mono-tartrate salt, and pharmaceutical composition thereof.

### Background of the Invention

Tofacitinib,3-{(3R,4R)-4-methyl-3-[methyl-(7H-pyrrolo[2,3-d]pyrimidin-4-yl)-amino]-piperidin-1-yl} 3-oxo-propionitrile, having the following formula: 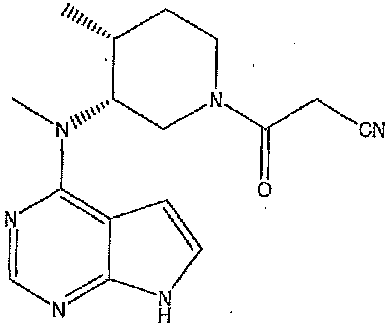 is under investigation as an inhibitor of protein kinases, such as the enzyme Janus Kinase 3 ("JAK3"). It has also been investigated as an immunosuppressive agent for the therapy of several conditions such as organ transplants, xeno transplantation, lupus, multiple sclerosis, rheumatoid arthritis, psoriasis, Type I diabetes and complications from diabetes, cancer, asthma, atopic dermatitis, autoimmune thyroid disorders, ulcerative colitis, Crohn's disease, Alzheimer's disease, leukemia and other indications, where immunosuppression would be desirable (see WO 03/048126).

Tofacitinib, as well as certain pharmaceutically acceptable salts thereof, is described in WO 01104.2246 and WO 02/096909. WO 03/048162 describes crystalline and amorphous forms of the mono-citrate salt of Tofacitinib. WO 12/135338 describes processes for preparing certain other Tofacitinib salts.

The present invention relates to salts of Tofacitinib, in particular, amorphous Tofacitinib mono-tartrate.

Solid state properties of Tofacitinib salts can be influenced by controlling the conditions under which the salts, e.g., Tofacitinib mono-tartrate, are obtained in solid form.

Polymorphism, the occurrence of different crystal forms, is a property of some molecules and molecular complexes. A single molecule may give rise to a variety of polymorphs having distinct crystal structures and physical properties like melting point, thermal behaviors (e.g. measured by thermogravimetric analysis - "TGA", or differential scanning calorimetry- "DSC"), X-ray diffraction pattern, infrared absorption fingerprint, and solid state NMR spectrum. One or more of these techniques may be used to distinguish different polymorphic forms of a compound.

Different salts and solid state forms of an active pharmaceutical ingredient may possess different properties. Such variations in the properties of different salts and solid state forms may provide a basis for improving formulation, for example, by facilitating better processing or handling characteristics, improving the dissolution profile, or improving stability and shelf-life. These variations in the properties of different salts and solid state forms may also provide improvements to the final dosage form, for instance, if they serve to improve bioavailability. Different salts and solid state forms of an active pharmaceutical ingredient may also give rise to a variety of polymorphs or crystalline forms, which may in turn provide additional opportunities to assess variations in the properties and characteristics of a solid active pharmaceutical ingredient.

Discovering new salts and solid state forms of a pharmaceutical product can provide materials having desirable processing properties, such as ease of handling, ease of processing, storage stability, ease of purification or as desirable intermediate crystal forms that facilitate conversion to other polymorphic forms. New salts and solid state forms of a pharmaceutically useful compound can also provide an opportunity to improve the performance characteristics of a pharmaceutical product. It enlarges the repertoire of materials that a formulation scientist has available for formulation optimization, for example by providing a product with different properties, e.g., better processing or handling characteristics, or improved shelf-life. For at least these reasons, there is a need for additional salts and solid state forms of Tofacitinib, in particular there is a need for salts and solid state forms that have improved solubility.

### Summary of the Invention

The present invention provides salts of Tofacitinib, particularly amorphous Tofacitinib mono-tartrate, and pharmaceutical compositions containing them.

The present invention also encompasses the use of the amorphous Tofacitinib mono-tartrate salt for the preparation of other Tofacitinib salts, in particular the mono-citrate salt, solid state forms and/or pharmaceutical compositions thereof

The present invention also provides a process for preparing Tofacitinib salts, in particular a mono-citrate salt, by preparing the amorphous Tofacitinib mono-tartrate salt and converting it to another Tofacitinib salt, preferably, the mono-citrate.

The present invention also encompasses the amorphous Tofacitinib mono-tartrate salt described herein for use as medicaments, particularly for the treatment of cancer.

The present invention further provides a pharmaceutical composition comprising the amorphous Tofacitinib mono-tartrate salt, and at least one pharmaceutically acceptable excipient, for use as medicaments, particularly for the treatment of cancer. Processes for preparing the above pharmaceutical compositions are also provided.

The present invention also provides a method of treating a cancer, particularly a cancer mediated by a protein kinase, such as the enzyme Janus Kinase 3 ("JAK3"), the method comprising administering a therapeutically effective amount of amount of the amorphous Tofacitinib mono-tartrate salt or at least one of the above pharmaceutical compositions to a person suffering from cancer or in need of the treatment.

### Brief Description of the Drawings

Figure 1 shows an ¹H-NMR spectrum of Tofacitinib mono-tartrate.
Figure 2 shows an Fourier Transform Infrared (FTIR) spectrum of Tofacitinib mono-tartrate.
Figure 3 shows an X-ray powder diffractogram of Tofacitinib mono-tartrate.

### Detailed Description of the Invention

The present invention encompasses amorphous Tofacitinib mono-tartrate.

In some embodiments, Tofacitinib mono-tartrate according to the invention is substantially free of any other salts of Tofacitinib, and solid state forms according to the invention are substantially free of any other polymorphic forms, or of specified polymorphic forms of Tofacitinib. In any embodiment of the present invention, by "substantially free" is meant that the salts and solid state forms of the present invention contain 20% (w/w) or less, 10% (w/w) or less, 5% (w/w) or less, 2% (w/w) or less, particularly 1% (w/w) or less, more particularly 0.5% (w/w) or less, and most particularly 0.2% (w/w) or less of any other salts and polymorphs, respectively, or of a specified polymorph of Tofacitinib. In other embodiments, the salts, e.g., Tofacitinib mono-tartrate.
and solid state forms thereof according to the invention can contain from 1% to 20% (w/w), from 5% to 20% (w/w), or from 5% to 10% (w/w) of any other salts, or solid state forms, or of a specified polymorph of Tofacitinib.

The new Tofacitinib salt of the present invention has advantageous properties selected from at least one of: chemical purity, flowability, solubility, dissolution rate, morphology or crystal habit, stability- such as thermal and mechanical stability to polymorphic conversion, stability to dehydration and/or storage stability, low content of residual solvent, a lower degree of hygroscopicity, flowability, and advantageous processing and handling characteristics such as compressibility, and bulk density. In particular, the Tofacitinib salt of the present invention has higher solubility in comparison to Tofacitinib mono-citrate. This property expands the methods of formulating that can be used. Furthermore, the amorphous form of the Tofacitinib mono-tartrate salt of the present invention is polymorphically stable, i.e. does not convert to other crystalline forms.

A crystal form may be referred to herein as being characterized by graphical data "as depicted in" a Figure. Such data include, for example, powder X-ray diffractograms and solid state NMR spectra. The skilled person will understand that such graphical representations of data may be subject to small variations, *e.g.,* in peak relative intensities and peak positions due to factors such as variations in instrument response and variations in sample concentration and purity, which are well known to the skilled person. Nonetheless, the skilled person would readily be capable of comparing the graphical data in the Figure. herein with graphical data generated for an unknown, crystal form and confirm whether the two sets of graphical data are characterizing the same crystal form or two different crystal forms. A crystal form of a Tofacitinib salt referred to herein as being characterized by graphical data "as depicted in" a Figure will thus be understood to include any crystal forms of the Tofacitinib salt characterized with the graphical data having such small variations, as are well known to the skilled person, in comparison with the Figure.

As used herein, the term "isolated" in reference to any of Tofacitinib salts or solid state forms thereof of the present invention corresponds to Tofacitinib salt or solid state form thereof that is physically separated from the reaction mixture, where it is formed.

As used herein, unless stated otherwise, the XRPD measurements are taken using copper Kα radiation wavelength 1.5406 A.

A thing, *e.g.,* a reaction mixture, maybe characterized herein as being at, or allowed to come to "room temperature, often abbreviated "RT." This means that the temperature of the thing is close to, or the same as, that of the space, *e.g.,* the room or fume hood, in which the thing is located. Typically, room temperature is from about 20°C to about 30°C, or about 22°C to about 27°C, or about 25°C.

A process or step may be referred to herein as being carried out "overnight." This refers to a time interval, *e.g.,* for the process or step, that spans the time during the night, when that process or step may not be actively observed. This time interval is from about 8 to about 20 hours, or about 10-18 hours, typically about 16 hours.

As used herein, the term "reduced pressure" refers to a pressure of about 10 mbar to about 50 mbar.

As used herein, the terms "vol." or "volume" can be used to refer to ml per gram of the corresponding Tofacitinib. For example, a statement that 0.5 g of Tofacitinib is dissolved in ten volumes of a Solvent X would be understood to mean that the 0.5 g of Tofacitinib was dissolved in 5 ml of Solvent X.

The present invention comprises a novel salt of Tofacitinib, in particular: tartrate.

The above salt can be isolated.

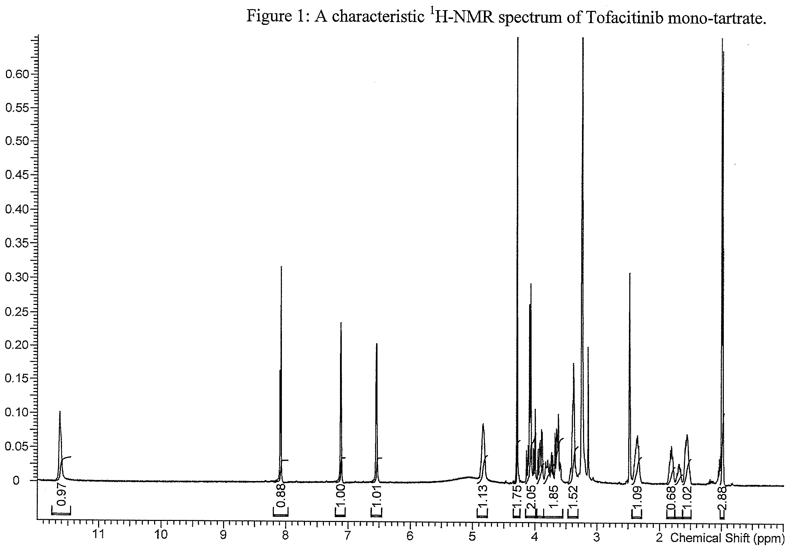

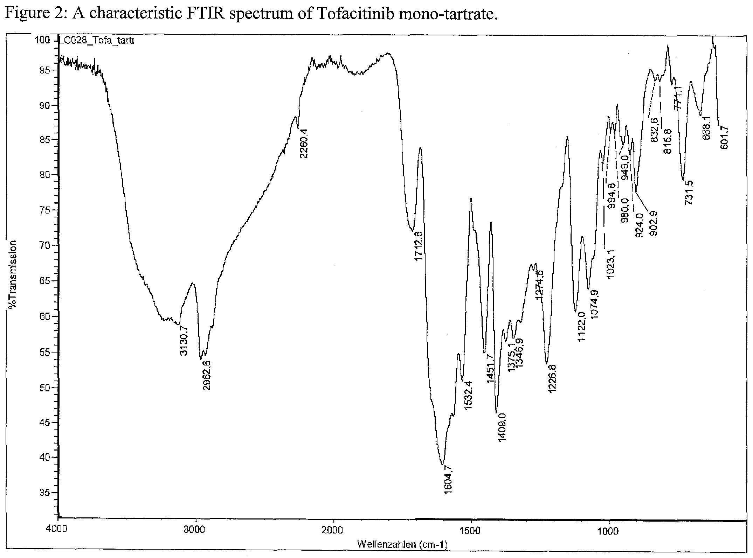

In one embodiment, the present invention comprises Tofacitinib mono-tartrate salt. The Tofacitinib mono-tartrate salt can be characterized by data selected from: an ¹H-NMR spectrum substantially as depicted in Figure 1, an FTIR spectrum with peaks at: 2963, 1713, 1605, 1532, 1452, 1409, 1227, 1122, 1075, 903, 732 cm⁻¹; an FTIR spectrum substantially as depicted in Figure 2; and combinations thereof.

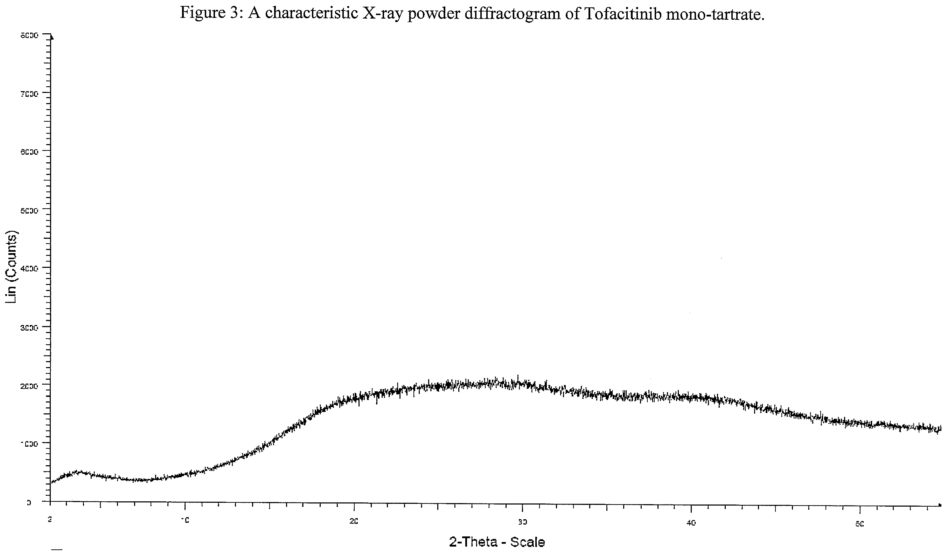

The Tofacitinib mono-tartrate is in amorphous form. The amorphous form of Tofacitinib mono-tartrate salt can be characterized by an X-ray powder diffraction pattern substantially as depicted in Figure 3.

The Tofacitinib salt of the present invention may be prepared by a process comprising: dissolving Tofacitinib in a suitable solvent to form a solution; and adding the corresponding acid to the solution.

The above described Tofacitinib mono-tartrate salt can be used to prepare other salts of Tofacitinib, in particular the mono-citrate salt, as well as solid state forms and/or pharmaceutical formulation thereof.

The present invention encompasses a process for preparing Tofacitinib salts, in particular a mono-citrate salt, the process comprising preparing the amorphous Tofacitinib mono-tartrate salt by the processes of the present invention, and converting that salt to said other Tofacitinib salt, preferably, Tofacitinib mono-citrate. The conversion can be done, for example, by a process comprising basifying a solution of the above described Tofacitinib salt to produce Tofacitinib base, and redacting the obtained Tofacitinib base with a suitable acid, to obtain the corresponding salt. Suitable acids include citric acid

The Tofacitinib mono-tartrate salt and/or solid state forms thereof of the present invention can also be used as a medicament.

The present invention further encompasses 1) a pharmaceutical composition comprising the amorphous Tofacitinib mono-tartrate as described above, and at least one pharmaceutically acceptable excipient; and 2) the use of . the above-described amorphous Tofacnmib mono-tartrate salt in the manufacture of a pharmaceutical composition, and 3) a method of treating a person suffering from cancer, comprising administration of an effective amount of a pharmaceutical composition comprising the Tofacitinib mono-tartrate salt described herein.

The above pharmaceutical compositions can be prepared by a process comprising combining amorphous Tofacitinib mono-tartrate salt with at least one pharmaceutically acceptable excipient.

. The Examples are set forth to aid in understanding the invention but are not intended to, and should not be construed to limit its scope in any way. Examples marked with an asterisk (*) relate to reference examples.

### Nuclear magnetic resonance (NMR) spectroscopy

Instrument: Varian Mercury 400 Plus NMR Spectrometer, Oxford AS, 400 MHz

**Infrared Spectroscopy**

| | |
|---|---|
| Instrument: | Thermo Nicolet, Avatar 330 FT-IR. Smart Endurance Diamond-ATR |
| Software: | Omnic Vers. 6.1a |

### X-Ray Powder Diffraction

The sample was analyzed on a D8 Advance X-ray powder diffractometer (Bruker-AXS, Karlsruhe, Germany). The sample holder was rotated in a plane parallel to its surface at 20 rpm during the measurement. Further conditions for the measurements are summarized in the table below. The raw data were analyzed with the program EVA (Bruker-AXS, Germany).

| | standard measurement |
|---|---|
| radiation | Cu K_{α} (λ =1.5406 Å) |
| source | 38kV / 40mA |
| detector | Vantec |
| detector slit | variable |
| divergence slit | v6 |
| antiscattering slit | v6 |
| 2θ range /° | 2 ≤ 2θ ≤ 55 |
| step size/° | 0.017 |

### Examples

### Reference example: Preparation of Tofacitinib

Tofacitinib citrate (24.3 g; 48.2 mmol) and dichloromethane (DCM) (200 ml) were mixed and stirred in a 21 Erlenmeyer flask. A10 % (w/v) aqueous potassium carbonate solution (305 ml) was added, and the mixture was stirred for an additional 5 min at RT and then transferred into a separation funnel. The organic phase was separated and the aqueous phase was extracted with an additional 200 ml DCM. The combined organic extract was washed with saturated sodium hydrogen carbonate (100 ml), then dried over sodium sulphate, followed by filtration and evaporation of the solvent. The residue was then dissolved in 150 ml methanol and concentrated. The resulting residue was dried at 50°C/100 mbar for 4 h, followed by 65°C/20 mbar O/N to produce Tofacitinib base (15.0 g, 99.2 %). The starting Tofacitinib citrate can be prepared for example according to the process described in "Mild and Efficient DBU-Catalyzed Amidation of Cyanoacetates" Org. Lett., 2009, Vol. 11, No. 9.

### Example 1: Preparation of Tofacitinib mono-tartrate salt

Tofacitinib (As produced in the reference example) (0.25 g) was dissolved in 2.5 ml of ethanol. L-Tartaric acid (0.18 g) was added and the resulting suspension was heated to reflux until a clear solution was obtained, stirred for 5 min and allowed to cool down slowly to room temperaure with stirring. A precipitate formed and was isolated by filtration, and dried under reduced pressure to yield 0.32 g of Tofacitinib mono-tartrate as a white powder.

### Example 2: Preparation of Tofacitinib mono-malate salt *

Tofacitinib (0.10 g) (As produced in the reference example) was dissolved in 1.0 ml of ethanol. D, L-Malic acid (0.043 g) was added and the resulting suspension was heated to reflux, until a clear solution was obtained. Then, the solution was allowed to cool down to room temperature and stirred for 3 h at room temperature. Diethylether (2 mL) was added in one portion and the resulting precipitate was collected by filtration. The collected precipitate was dissolved in 2 ml of methanol and the solution was concentrated. The thus-obtained solid residue was dried under vacuum to yield 0.13 g of Tofacitinib mono-malate as a white powder.

### Example 3: Preparation of Tofacitinib mono-oxalate salt *

Tofacitinib (0.10 g) (As produced in the reference example)was dissolved in 1.0 ml of ethanol. Oxalic acid dihydrate (0.040 g) was added in portions. This suspension was then heated to reflux, until a clear solution was obtained, and then allowed to cool down to room temperature and stirred for 3 h at room temperature. The volatiles were distilled off and the crude product was dissolved in 2 ml of isopropanol and concentrated. The thus-obtained solid residue was dried under vacuum to yield 0.13 g of Tofacitinib mono-oxalate as a white powder.

### Example 4: Solubility of Tofacitinib salts

The solubility of Tofacitinib salts was investigated in 0.1 N HCl (pH 1.2), 20 mM sodium acetate (pH 4.5) and 50 mM potassium dihydrogen phosphate (pH 6.8). For each salt, 250 mg of the salt was weighed into a test tube and 2.5 ml solvent was added. After mixing, the suspension was stirred for 1 h at room temperature. A sample was withdrawn, filtered and analyzed by HPLC with UV-detection (λ=290 nm). Quantification of Tofacitinib was based on a calibration curive, which was established prior to the solubility test. For Tofacitinib tartrate, Tofacitinib malate and Tofacitinib oxalate, clear solutions were obtained, indicating that saturation was not yet achieved. Therefore, for these salts, a new experiment was performed by mixing 250 mg solid substance with 2.5 ml solvent. The results are summarized in the table below.

| **compound** | **solid state** | **batch no.** | **solubility [mg/ml] pH 1.2** | **pH 4.5** | **pH 6.8** |
|---|---|---|---|---|---|
| TOF base | amorphous | LC229 | 36,7 | 6,9 | 2,1 |
| TOF-citrate | crystalline | LC125_Pr2 | 29,4 | 9,4 | 11,6 |
| TOF-citrate | amorphous | LC190* | 29,7 | 3,7 | 4,1 |
| TOF-citrate | amorphous | LC259** | N/D | N/D | 5,4 |
| TOF-tartrate | amorphous | LC242 | 381 | 324 | 353 |
| TOF-malate | amorphous | LC243 | 361 | 364 | 377 |
| TOT-oxalate | amorphous | LC247 | 375 | 329 | 350 |

| | | | | | |
|---|---|---|---|---|---|
| *: LC190 prepared by milling of Tofacitinib base with citric acid (400 rpm, 60 min) **: LC259 prepared by milling of Tofacitinib base with citric acid (400 rpm, 2x60 min) | | | | | |

## Claims

1. Amorphous Tofacitinib mono-tartrate salt.

2. A pharmaceutical composition comprising amorphous Tofacitinib mono-tartrate salt according to claim 1 and at least one pharmaceutically acceptable excipient.

3. A process for preparing the pharmaceutical composition of claim 2, comprising combining amorphous Tofacitinib mono-tartrate salt according to claim 1 with at least one pharmaceutically acceptable excipient.

4. Use of amorphous Tofacitinib mono-tartrate salt according to claim 1 to prepare Tofacitinib mono-citrate salt.

5. A process for preparing a Tofacitinib mono-citrate salt comprising preparing amorphous Tofacitinib mono-tartrate salt according to claim 1 and converting it to Tofacitinib mono-citrate.

6. The process according to claim 5, wherein the converting is done by a process comprising basifying a solution of amorphous Tofacitinib mono-tartrate salt according to claim 1 to produce Tofacitinib base, and reacting the Tofacitinib base with citric acid, to obtain the Tofacitinib mono-citrate.

7. Amorphous Tofacitinib mono-tartrate salt according to claim 1 for use as a medicament.

8. Use of amorphous Tofacitinib mono-tartrate salt according to claim 1 in the manufacture of a pharmaceutical composition.

9. Amorphous Tofacitinib mono-tartrate salt according to claim 1, or a pharmaceutical composition thereof according to claim 2, for use in the treatment of cancer.

## Patentansprüche

1. Amorphes Tofacitinib-Monotartratsalz.

2. Pharmazeutische Zusammensetzung, umfassend amorphes Tofacitinib-Monotartratsalz gemäß Anspruch 1 und wenigstens einen pharmazeutisch verträglichen Hilfsstoff.

3. Verfahren zum Herstellen der pharmazeutischen Zusammensetzung gemäß Anspruch 2, umfassend Kombinieren von amorphem Tofacitinib-Monotartratsalz gemäß Anspruch 1 mit wenigstens einem pharmazeutisch verträglichen Hilfsstoff.

4. Verwendung von amorphem Tofacitinib-Monotartratsalz gemäß Anspruch 1 zum Herstellen von Tofacitinib-Monocitratsalz.

5. Verfahren zum Herstellen eines Tofacitinib-Monocitratsalzes, umfassend Herstellen von amorphem Tofacitinib-Monotartratsalz gemäß Anspruch 1 und Umwandeln davon zu Tofacitinib-Monocitrat.

6. Verfahren gemäß Anspruch 5, wobei das Umwandeln durch ein Verfahren umfassend Basifizieren einer Lösung von amorphem Tofacitinib-Monotartratsalz gemäß Anspruch 1 zum Herstellen einer Tofacitinibbase und Umsetzen der Tofacitinibbase mit Citronensäure, um das Tofacitinibmonocitrat zu erhalten, durchgeführt wird.

7. Amorphes Tofacitinib-Monotartratsalz gemäß Anspruch 1 für die Verwendung als ein Medikament.

8. Verwendung von amorphem Tofacitinib-Monotartratsalz gemäß Anspruch 1 bei der Herstellung einer pharmazeutischen Zusammensetzung.

9. Amorphes Tofacitinib-Monotartratsalz gemäß Anspruch 1 oder pharmazeutische Zusammensetzung davon gemäß Anspruch 2 für die Verwendung bei der Behandlung von Krebs.

## Revendications

1. Sel de monotartrate de Tofacitinib amorphe.

2. Composition pharmaceutique comprenant le sel de monotartrate de Tofacitinib amorphe selon la revendication 1 et au moins un excipient pharmaceutiquement acceptable.

3. Procédé d'élaboration de la composition pharmaceutique selon la revendication 2, comprenant la combinaison du sel de monotartrate de Tofacitinib amorphe selon la revendication 1 avec au moins un excipient pharmaceutiquement acceptable.

4. Utilisation du sel de monotartrate de Tofacitinib amorphe selon la revendication 1 pour préparer le sel de monocitrate de Tofacitinib.

5. Procédé de préparation d'un sel de monocitrate de Tofacitinib comprenant la préparation du sel de monotartrate de Tofacitinib amorphe selon la revendication 1 et sa conversion en monocitrate de Tofacitinib.

6. Procédé selon la revendication 5, où la conversion est mise en oeuvre par un procédé comprenant la basification d'une solution de sel de monotartrate de Tofacitinib amorphe selon la revendication 1 pour produire du Tofacitinib basique, et la réaction du Tofacitinib basique avec de l'acide citrique pour obtenir le monocitrate de Tofacitinib.

7. Sel de monotartrate de Tofacitinib amorphe selon la revendication 1 pour utilisation comme médicament.

8. Utilisation de sel de monotartrate de Tofacitinib amorphe selon la revendication 1 dans la fabrication d'une composition pharmaceutique.

9. Sel de monotartrate de Tofacitinib amorphe selon la revendication 1, ou composition pharmaceutique de celui-ci selon la revendication 2, pour utilisation dans le traitement du cancer.
